Europäisches Patentamt

European Patent Office

Office européen des brevets

(11) Publication number: **0 345 938**
**A1**

# EUROPEAN PATENT APPLICATION

(21) Application number: 89304347.1

(51) Int. Cl.⁴: **A61F 2/08**

(22) Date of filing: 28.04.89

(30) Priority: 29.04.88 GB 8810222

(43) Date of publication of application:
**13.12.89 Bulletin 89/50**

(84) Designated Contracting States:
**ES**

(71) Applicant: **Seedhom, Bahaa Botros**
**30 Blenheim Terrace**
**Leeds West Yorkshire LS2 9HD(GB)**

(72) Inventor: **Seedhom, Bahaa Botros**
**30 Blenheim Terrace**
**Leeds West Yorkshire LS2 9HD(GB)**

(74) Representative: **Orr, William McLean et al**
**URQUHART-DYKES & LORD 5th Floor, Tower**
**House Merrion Way**
**Leeds West Yorkshire, LS2 8PA(GB)**

(54) **Mechanical fixation of prosthetic ligament.**

(57) There is disclosed a prosthetic ligament (20) made of woven implantable material and which can be introduced into a passage (11) formed in a pair of adjoining bones (12, 13) of a bone joint, there being enlarged entrances (14) at each end of passage (11) to receive bone plugs (14a) for closing the entrances after the ligament has been introduced into the passage and the entrances. The ligament (20, 30) comprises a main tubular portion (21) to be located in the passage (11) and having its axis extending generally longitudinally of the ligament, and end portions (25) each intended to be located in a respective entrance (14) and to project therefrom for securement to the respective bone (12, 13) by mechanical fixation. At least one of the end portions (25) comprises a tubular portion (26) which extends transversely of the longitudinal axis and which is open at each end so as to receive an elongate anchoring element (a cross piece of a staple 28 or an anchoring bar 31) of a mechanical fixation arrangement which is operative to make a mechanical fixation of the end portion to the respective bone, and there is also provided a strengthened section (27) in the form of a dense weave section which is adjacent to the transverse tubular portion (26) and which can therefore withstand tensile load which may be applied in use to the ligament.

FIG. 3

# MECHANICAL FIXATION OF PROSTHETIC LIGAMENT

This invention relates to the mechanical fixation of a prosthetic ligament and has been developed primarily, though not exclusively, with a view to providing improved initial and / or temporary mechanical fixation of a biologically anchored prosthetic ligament so as to permit early mobilisation.

Prosthetic ligaments are routinely used at present in many centres around the world to replace knee and ankle ligaments ruptured during sporting activities and motor vehicle accidents.

On of the greatest problems encountered in this field of prosthetic ligament is that of anchoring the device to the knee bones. Mechanical methods have been used, such as screws, toggles and bollards, and super glue, but these have a distinct disadvantage: they fatigue and / or fail, and further, they do not increase in strength with time.

Biological anchoring methods have been advocated and one in particular upon which this invention is based is a prosthetic ligament which is anchored to the bone by a method which benefits from both bone and tissue ingrowth, and which is disclosed in more detail in EPA 0126520, and which is shown herein in Figure 1 of the accompanying drawings. This known prosthetis is made of polyester in the form of an open-weave structure, and in the case of a prosthetic replacement for the anterior cruciate ligament (ACL), it has a substantial tubular section. It has other structural features in the sections protruding from the bones on either side, which are described in EPA 0126520, to facilitate implanting and anchoring the prosthesis to the bone.

In the case of the ACL reconstruction, the implant is anchored to the femur and tibia using bony dowels taken from the same site and replaced within the tubular structure of the implant so that, with time, bony ingrowth unites the host bone to the bone dowel, and thereby anchors the ligament firmly. Tissue ingrowth in the intra-articular link attaches to the site of anchoring in the intra-condylar cavity. It is important therefore that the patient should be immobilised in plaster until such a growth has taken place.

However, it is clear from the concept of anchoring the implant that initially the fixation using bone plugs as such is not adequate, if early mobilisation is required (e.g. in the case of athletes), and that the ligament may slip within the small annular space between the bone plug and the bone tunnel unless prevented mechanically in the initial stages and until such a time as bone and tissue ingrowth have occured to provide the firm anchoring which should, and does, increase with time.

To this end, developments of the known prosthesis have been proposed, with a view to improving the temporary mechanical fixation while tissue and bone ingrowth takes place. In one proposal, at one end the ligament tubular structure is closed by combining the fibres of both halves of the tubular structure and, as a result of so doing, the weave in the closed section is dense in the hope that this closure would be adequate to prevent the ligament from slipping around the bone plug. However, it appears from experimental work in the laboratory and from clinical results that such a dense section is not quite adequate and slip seems to occur in the case of patients who have been mobilised immediately or soon after surgery.

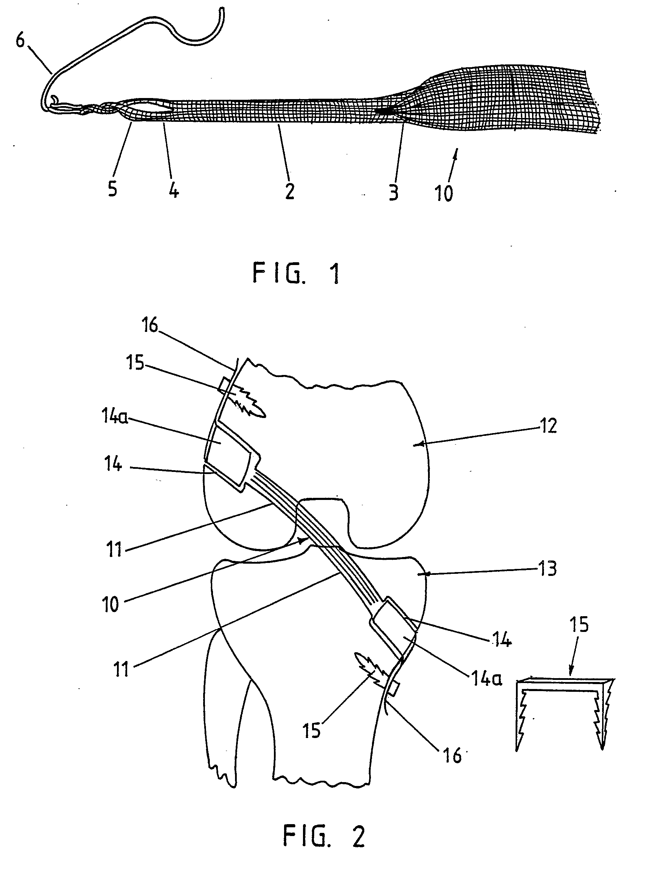

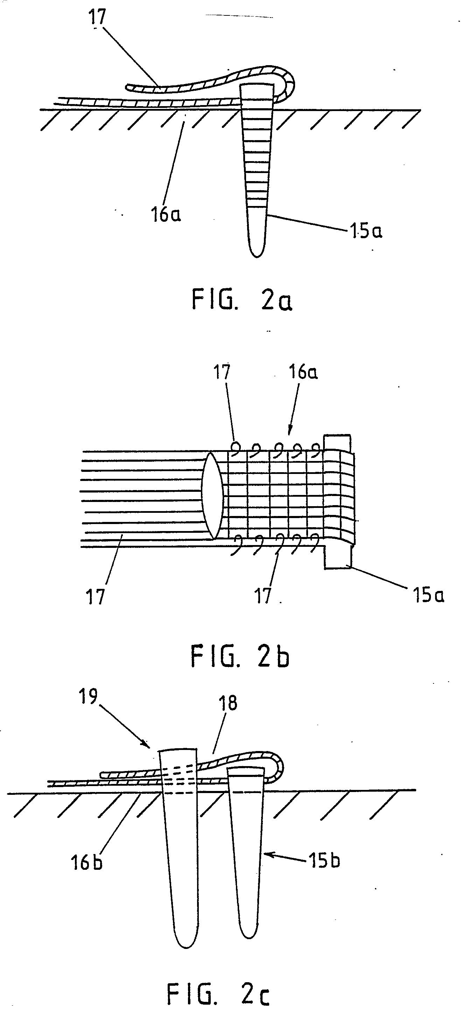

To overcome this problem, some surgeons have resorted to stapling of the implant to the bone by a single or double staple arrangement. Figure 2 of the accompanying drawings is a schematic illustration of a known surgical technique for applying temporary mechanical fixation to a prosthetic ligament used as a prosthetic replacement for the interior cruciate ligament (ACL), this technique being intended to allow early mobilisation of the patient and to hold the ligament in position during the period in which biological anchoring takes place by bone and tissue ingrowth through the prosthetic ligament. There is shown a prosthetic ligament, designated generally by reference 10, which may be generally similar to the ligament disclosed in EPA 0126520, which is introduced into a pre-drilled passage 11 taken through the adjoining bones 12, 13 of a knee joint. Enlarged entrance passages 14 are formed in the bones 12, 13, and bone plugs 14a are then extracted, which are subsequently replaced in position after the ligament 10 has been implanted, and as disclosed in more detail in EPA 0126520.

The temporary mechanical fixation is achieved by the use of one or more staples 15 driven into the bone 12 and over a projecting end 16 of the ligament 10. Figure 2 shows schematically the arrangement at the upper end of the ligament 10, and a similar arrangement may be provided at the lower end of the ligament.

Figures 2a and 2b show schematically the manner by which a single staple 15a can be driven into position into bone and through the end 16a of a ligament, and which end is then folded or looped-over the cross-piece of the staple 15a, and is then secured to the underlying portion of the ligament by sutures 17 looped along the overlapping edges.

Figure 2c shows an alternative known surgical technique, in which a ligament end 16b is first secured by a first staple 15b, is looped-over the cross-piece of the first staple 15b in a folded portion 18, and is then further secured by a second staple 19 which is driven downwardly into the bone after passing through the superposed layers (16b, 18) of the ligament.

It had been hoped that, with these temporary mechanical fixation techniques, pressure of the staple on the ligament would produce sufficient friction to prevent slip of the ligament when subjected to tension. However, it was found that this was not the case; the ligament does slip, and if the amount of movement is large, the ligament becomes slack within the joint and ceases to be efficient, if it does not become totally redundant.

The present invention therefore seeks to provide an improved structure of prosthetic ligament which is woven in such a way as to provide more reliable temporary mechanical fixation, when using at least one surgical staple, during the period in which biological anchoring takes place.

According to the invention there is provided a prosthetic ligament made of woven implantable material and which can be introduced into a passage formed in a pair of adjoining bones of a bone joint, there being enlarged entrances formed at each end of the passage to receive bone plugs for closing the entrances after the ligament has been introduced into the passage and the entrances, and the ligament comprising:

a main tubular portion to be located in said passage and having its axis extending generally longitudinally of the ligament; and,

end portions each intended to be located in a respective entrance and to project therefrom for securement to the respective bone by mechanical fixation:

in which at least one end portion of the ligament comprises:

a tubular portion which extends transversely of said longitudinal axis and which is open at each end so as to receive an elongate anchoring element of a mechanical fixation arrangement which is operative to make a mechanical fixation of the end portion to the respective bone; and

a strengthened section adjacent to the transverse tubular portion and arranged to withstand tensile load which may be applied in use to the ligament by the mechanical fixation arrangement.

Therefore, a prosthetic ligament according to the invention can be readily implanted, and reliable mechanical fixation can be applied which holds the ligament in position for a temporary period while biological anchoring takes place, thereby permitting early mobilisation of a patient which is now being considered desirable, especially for athletes and sportsmen and sportswomen. Thus, tensile loads which may be applied to the ligament during motion of the patient will be borne at least in part by the mechanical fixation, and which may result in the anchoring element applying tension to the adjacent portion of the ligament. However, by virtue of the provision of the adjacent strengthened section, these tensile loads can be borne without risk of tearing of the ligament taking place.

One preferred mechanical fixation arrangement comprises a staple which has its cross-piece received by the tubular portion so as to form said elongate anchoring element and the mechanical fixation can then be completed by driving the parallel limb of the staple into the underlying bone.

Preferably, the strengthened section is formed by providing a dense weave section.

The preferred weaving technique involved in the production of the ligament forms at least the main tubular portion as a mock leno weave, and the entire ligament (the main tubular portion, the transverse tubular portions and the dense weave sections) can be formed readily in the weaving operation.

To provide a particularly secure mechanical fixation, preferably the end portion is provided with a pair of transverse open-ended tubular portions, each intended to receive a cross-piece of a respective staple, so that a double staple mechanical fixation can take place. There will also be an associated strengthened section.

In a particularly preferred embodiment, the end portion is formed with an alternating series of transverse tubular portions and dense weave sections, whereby a double staple mechanical fixation can be obtained with the staples introduced at any desired position along the length of the end portion, by introduction into any selected ones of the tubular portions available. The dense weave sections on either side of the selected transverse tubular portions will be able to bear tensile loads applied in one direction or an opposite direction along the end portion.

For a double staple mechanical fixation, conveniently a first staple will be secured in position, leaving a projecting end of the ligament end portion which is folded back so as to overlie the first staple and then a second staple is secured in position in a tubular portion of the folded-over part so as to hold down overlapping layers of the end portion of the ligament.

Further, in the preferred embodiment, it is advantageous for at least one pouch to be formed (as disclosed in more detail in EPA 0126520), for receiving a respective bone plug for closing a respective entrance to the passage.

It is further preferred that the further end portion of the ligament is similarly provided with one or more transverse tubular portions and strengthened sections.

One preferred form of mechanical fixation arrangement for use with a prosthetic ligament according to the invention comprises, as referred to above, a U-shaped staple of the type used in implant surgery. However, other types of mechanical fixation arrangement may be employed. Thus, in a further preferred arrangment, a mechanical fixation of one end portion of the prosthetic ligament may be achieved by the use of a bar (or pin) which forms said elongate anchoring element and which is introduced into the respective transverse tubular portion, and a staple can then be driven into the underlying bone, and immediately adjacent to the bar. The staple will be positioned on the side of the bar which faces the direction in which ligament tension will be applied in service, so that the end portion of the ligament can be securely held in position by interengagement between the staple (which is driven into the bone) and the bar.

It has been found that this arrangement enables ligament tension to be more readily maintained after the staple has been driven into the bone, than may be possible when the staple has its cross piece taken through the tubular portion, as in the first described preferred arrangement.

Thus, the bar can be readily introduced into its tubular portion, and the staple can be applied over the ligament with its parallel spikes bridging the ligament which is located therebetween. The staple can therefore be readily slid longitudinally of the ligament to engage the bar and to apply any required initial tension to one end portion of the ligament (assuming the other end portion has already been anchored), and then the staple can be driven into the underlying bone.

The bar may be formed in such a way as to have cooperative engagement with the staple. For example, the bar ends may be inturned so as to hold the staple spikes, or the ends of the cross piece.

The bar may also be formed with a rebate onto which the end portion of the ligament can be laid.

To further strengthen the mechanical fixation of the ligament, the free end of the ligament may be folded over the bar, after insertion into its tubular portion, and the staple may then be driven downwardly into the bone and engaging over both the folded over end, and also the underlying portion of the ligament.

In a further strengthened arrangement, a bar can be taken through one tubular portion and a first staple can have its cross piece taken through an adjacent tubular portion, and has its spikes driven into the bone. The free end of the ligament can then be folded back over the cross piece of the first staple, and a second staple can then be driven down over the two layers of ligament and adjacent to the bar. This therefore provides a particularly strong retention of the ligament end portion, by virtue of the double staple fastening and also the folded over end of the ligament. The free end of the ligament may be reverse folded to extend under the second staple so as to form three superposed layers which will be trapped below the cross piece of the second staple, to provide a still more secure arrangement.

If it may be required to carry out additional ligament reconstruction, such as extra-articular reconstruction of ligaments or tissue e.g. medial collateral ligaments of the knee or lateral extra-articular reinforcement, the ligament may be formed with an additional length which will project from the passage in one of the bones, and which can then be externally applied over the bone joint and secured in position.

To facilitate entry of the elongate anchoring element into its tubular portion, which may be difficult on occasions for a surgeon wearing gloves which may be wet, means may be provided to facilitate temporary enlargement of the entrance mouth to the tubular portion at one or both ends thereof. This may comprise a cord or draw-string looped through the tubular portion and taken through a guide, sich as a slip knot and which can be tightened so as to draw the ends of the tubular portion towards each other and thereby open up the mouths at the ends. The cord may be cut subsequently after introduction of the elongate anchoring element.

The invention will now be described in more detail, by way of example only, with reference to the accompanying drawings in which:

Figure 1 is a schematic illustration of a known prosthetic ligament as disclosed in EPA 0126520, and to which the invention may be applied;

Figure 2 is a schematic illustration of a known mechanical fixation technique employed with the ligament shown in Figure 1;

Figure 2a, 2b and 2c show further known mechanical fixation techniques;

Figure 3 is a perspective illustration of an embodiment of prosthetic ligament according to the invention;

Figures 4a to 4d are detailed illustrations of the arrangement at one or both of the end portions of the ligament shown in Figure 3;

Figure 5 is a schematic plan view of a further embodiment of prosthetic ligament according to the invention;

Figure 6 is a detail side view showing mechanical fixation applied to one end portion of the ligament shown in Figure 5;

Figure 7 is a detail view, similar to Figure 6, showing an alternative means for mechanical fixation;

Figure 8 is a detail plan view corresponding to Figure 7;

Figure 9 is a side view, corresponding to Figure 7, showing a still further mechanical fixation arrangment;

Figure 10 is a detail plan view corresponding to Figure 9;

Figure 11 is a detail perspective view showing one preferred profile arrangment of anchoring bar for use with the mechanical fixation arrangements shown in Figures 7 to 10;

Figure 12 is a detail illustration of an alternative profile of the anchoring bar;

Figure 13 is a detail view showing a detachable guide head provided on one end of the anchoring bar;

Figure 14 is a schematic plan view showing a cord arrangement for opening up a transversely extending tubular portion in which the anchoring element can be received; and,

Figure 15 is a detail view in schematic form of an extension piece which may be attached to, or formed integrally during the formation of the embodiments of prosthetic ligament as shown in Figures 3 or 5.

Referring first to Figure 1 of the drawings, the known prosthetic ligament is designated by reference 1 and comprises an elongate strip of open weave polyester. This fabric is terylene and is a mock leno weave comprising a warp of 550 decitex polyester yarn and a weft of twisted polyester yarn. The holes formed in the woven strip are about 0.1 by 0.2 cm, and there are 14 or 15 holes across the width of the strip, and 20 holes per square cm.

The ligament is in the form of a tube 2 which has a slit 3 at one open end of the ligament extending to the open end edge of the ligament, and a slit 4 at the other closed end of the ligament. The end of the slit 4 remote from the centre of the tube 2 is sealed by a row of stitching 5 or the like, to form a pouch. A cord 6 is attached to the end of the ligament to pull the ligament through the passages formed in the bones.

The known surgical techniques for implanting the ligaments 10, and applying temporary mechanical fixation to each end thereof, have been described in more detail in the introductory part of the specification with reference to Figure 2 and Figures 2a, 2b and 2c.

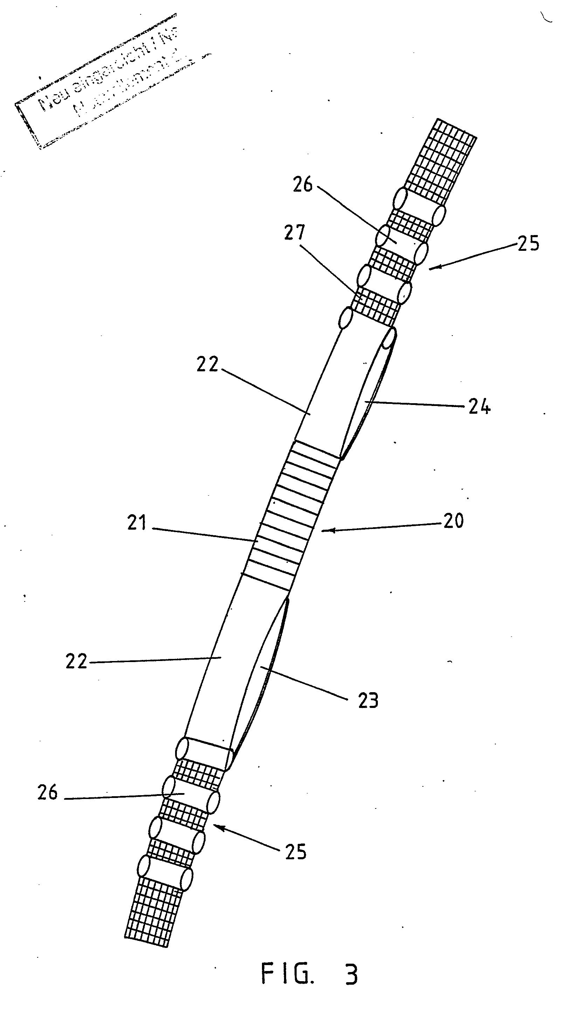

An improved prosthetic ligament will now be described with reference to Figure 3, which shows a preferred embodiment designated generally by reference 20, and which enables more reliable mechanical fixation to be achieved at at least one projecting end portion of the ligament, using one or more surgical staples. In the illustrated embodiment, each projecting end portion is similarly provided, and can use a double staple arrangement, but it should be understood that the invention, in its simplest form, will include mechanical fixation of one end portion only using a single staple, if required.

The prosthetic ligament 20 is made of woven implantable material and can be introduced into passage 11 formed in a pair of adjoining bones 12 and 13 of a bone joint, as shown in Figure 2, there being enlarged entrances 14 to receive bone plugs 14a for closing the entrances 14 after the ligament 10 has been introduced into the passage 11 and the entrances 14.

The ligament 20 comprises a main tubular portion which is intended to be located in the passage 11, and has its axis extending longitudinally of the ligament. The main tubular portion has a central tubular section 21, and adjoining sections 22 each formed with a lateral entrance opening 23 whereby a pouch is defined into which a bone plug 14a can be introduced prior to or during implantation of the ligament 20.

End portions designated generally by reference 25 adjoin the main tubular portion, and each end portion is intended to be located in a respective one of the entrances 14 and to project therefrom for securement to the respective bone by mechanical fixation, using surgical staples which may be similar to the staples 15 described above with reference to Figures 2, 2a and 2b.

The end portions 25 of the ligament each have at least one tubular portion 26 which extends transversely of the longitudinal axis of the main portion and which is open at each end, so as to receive a cross-piece of a surgical staple to be used for mechanical fixation of the end portion to the respective bone. There is also at least one strengthened section 27 adjacent to the transverse tubular portion 26, which is arranged to withstand tensile load which may be applied in use to the ligament 20 by the mechanical fixation via the staple.

The ligament 20 can therefore be readily implanted, and reliable mechanical fixation can be applied which holds the ligament in position for a temporary period while biological anchoring takes place, thereby permitting early mobilisation of a patient.

As can be seen from the preferred embodiment, an alternating series of transverse tubular portions 26 and strengthened sections 27 are provided, whereby a double staple mechanical fixation can be obtained with the staples being introduced at any desired positions along the length of the end portions, by introduction into any selected ones of the tubular portions available. The strengthened sections 27 are preferably formed as dense weave sections, and it can be seen that there is a dense weave section on either side of each transverse tubular portion 26, whereby tensile loads applied in service in either direction from the cross-piece of the staple can be readily borne by the dense weave sections, thereby minimising the risk of tension failures.

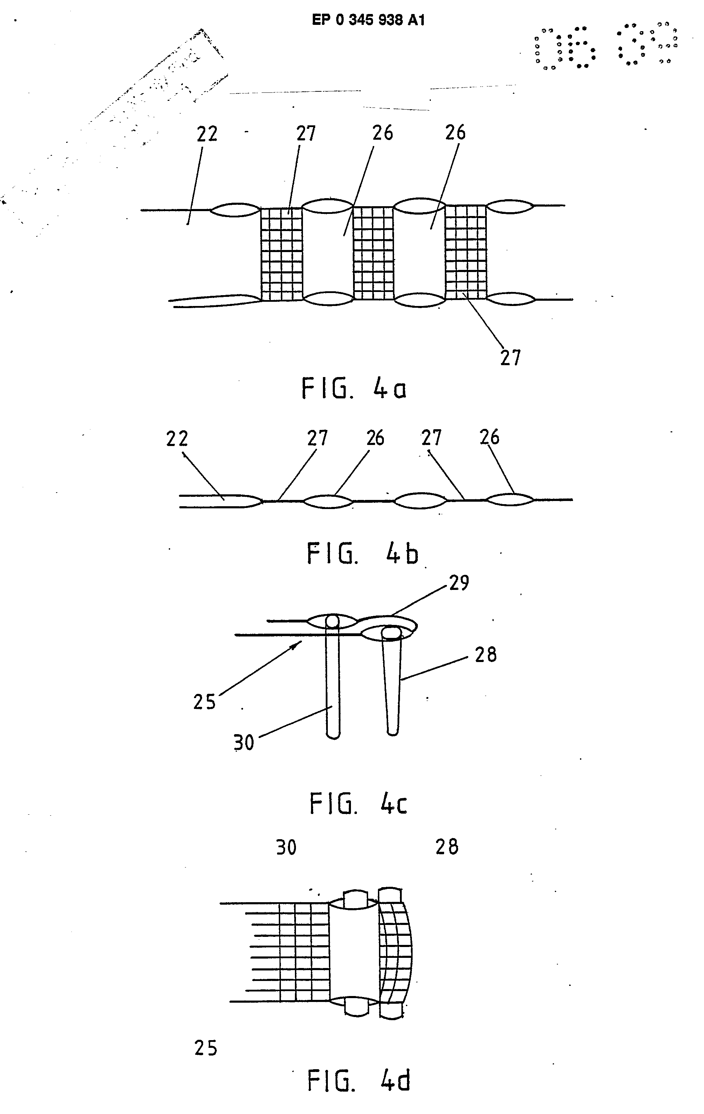

Figure 4a is a plan view of one of the end portions 25, which clearly shows the alternating series of transverse open-ended tubular portions 26 and dense weave sections 27, immediately adjacent to one of the bone pouch ends of the main portion, and Figure 4b is a schematic side view of the arrangement shown in Figure 4a.

Figure 4c and 4d are respectively side views and plan views of a double staple mechanical fixation, in which a first staple 28 is secured in position, leaving a projecting end 29 of the ligament end portion which is folded-back so as to overlie the first staple 28, and then a second staple 30 is secured in position in the folded-over part 29 and is then driven downwardly into the bone so as to hold-down overlapping layers of the end portion of the ligament.

The implant is stapled to the bone by the double stapling system, as described above with reference to Figures 4c and 4d. Thus, one staple 28 is passed between the two halves of the section between two dense sections and is stapled to the bone. The ligament is next folded over the staple 28 and is stapled to the bone by another staple 30 which passes between the two halves of the next double split section. Thus, the first staple 28 has between it and the bone one layer of ligament weave, whereas the second staple 30 has three layers.

Any tension applied to the ligament would be carried, thereby, by the dense section and not just by friction between the implant and the bone, as in the known surgical techniques.

The mechanical fixation which can be achieved using one or more staples, applied to a ligament as shown in the preferred embodiment, has been found in laboratory tests to be much more effective as a mechanical anchoring system, than all other stapling methods previously used and reviewed herein, or that obtainable by the use of the dense section by itself. The ligament is therefore particularly recommended for use in the case of athletes who desire early mobilisation.

With the inclusion of two end sections 25 (one at either end of the main portion of the ligament), each having alternating dense and double split sections, the total length of the ligament will have to be considered carefully, to take into account the different sizes of the knees (the preferred use of the ligament) of the patient receiving a prosthetic ligament replacement. However, as indicated above, the invention in its broadest aspect relates to a prosthetic ligament of woven material having one end portion only 25 which is provided with at least one transverse tubular portion 26 and adjacent strengthened section 27, for use in conjunction with a single staple.

A preferred method of weaving of the implant 20 will now be described. The prosthetic ligament or implant 20 has, at either end, a section of alternating densely-woven section (closing the tube), and another where the tubular section is split open at both sides of the implant, and where the two halves of this section are either woven in mock leno (as the main body of the ligament), or one is woven in mock leno and the other one is weftless.

The embodiment described above with reference to Figures 3 and 4 provides one form of mechanical fixation arrangement, having a U-shaped staple 15 of the type used in implant surgery. However, other types of mechanical fixation arrangement may be employed, as will be described in detail below, with respect to a further preferred embodiment of the invention, in which mechanical fixation of the or each end portion of a prosthetic ligament may be achieved by the use of a bar or pin which forms an elongate anchoring element to be introduced into a respective transverse tubular portion. A staple can then be driven into the underlying bone, and immediately adjacent to the bar, and positioned on the side of the bar which faces the direction in which ligament tension will be applied in service.

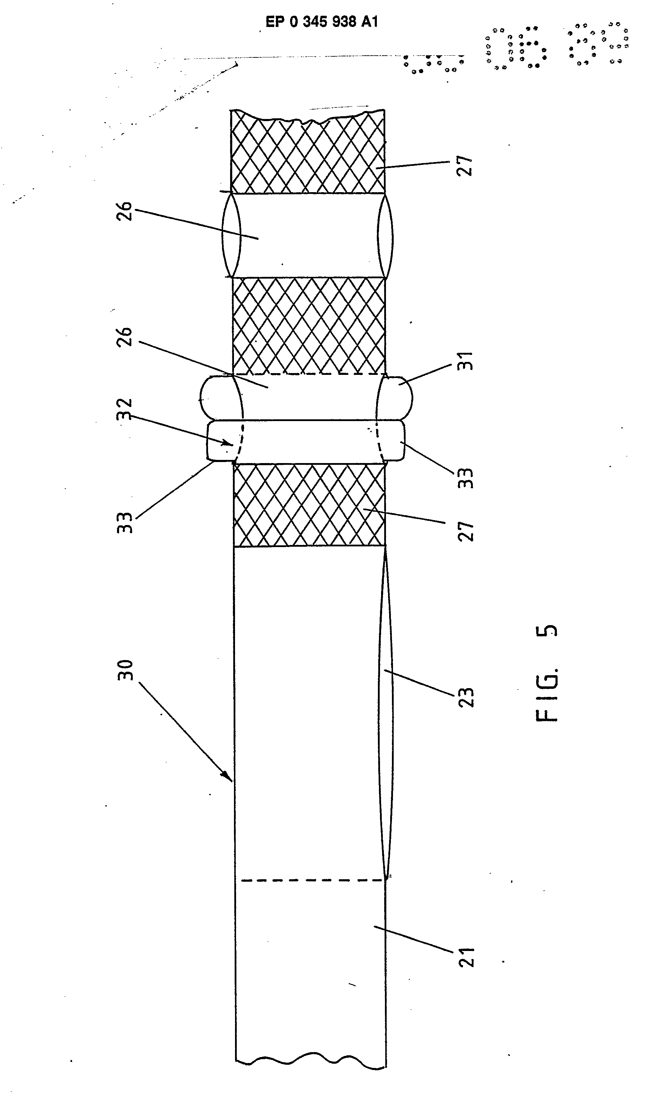

Referring now to Figure 5, there is shown a prosthetic ligament designated generally by reference 30, and which is very similar in construction to the ligament 20 shown in Figure 3. Corresponding parts are therefore designated by the same reference numerals, and will not be described in detail again.

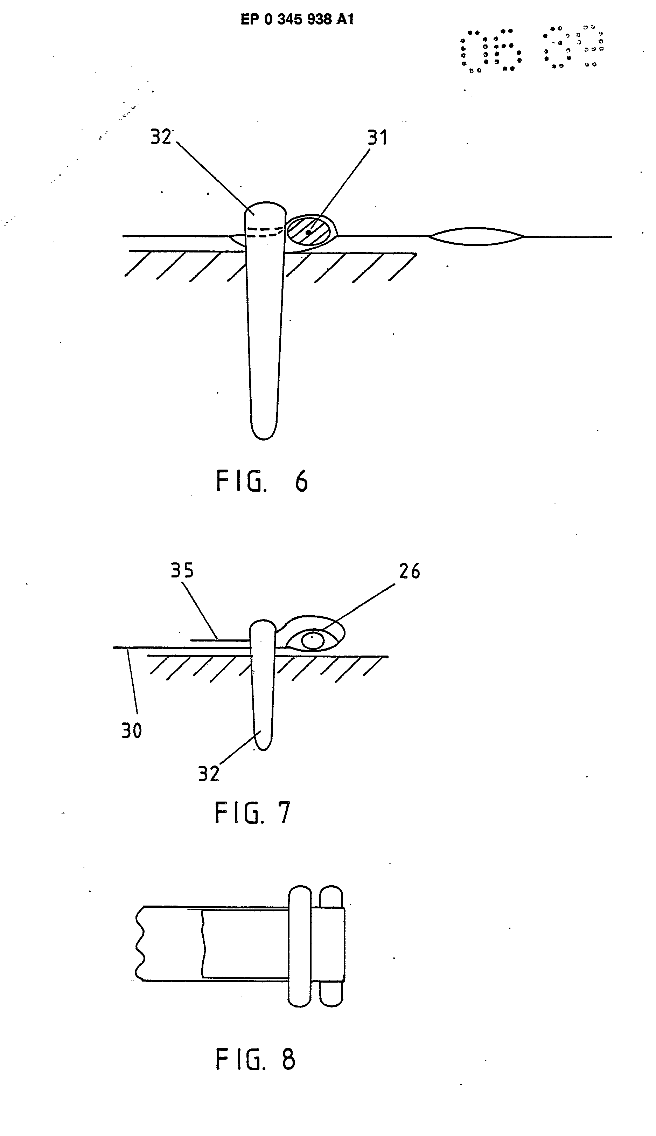

An alternative mechanical fixation arrangement is provided, as can be seen from Figures 5 and 6, in which an elongate anchoring element in the form of bar 31 is introduced with a relatively loose fit into transverse tubular portion 26, in place of the cross piece of a U-shaped staple, as in the first described embodiment. U-shaped staple 32 is then driven into the underlying bone, as shown in Figure 6, and with its two parallel spikes 33 fitting over or bridging the underlying portion of the ligament 30. The staple 32 engages with the bar 31, at least at the ends of the bar, and in the illustrated embodiment throughout the length of the bar, and this provides secure anchoring of the end portion of the ligament.

It has been found that this arrangement enables ligament tension to be more readily maintained after the staple 32 has been driven into the bone, and may be possible when the staple has its cross piece taken through tubular portion 26 as in the first described preferred arrangement.

The staple 32 can be applied over the ligament with its parallel spikes bridging the ligament which is located therebetween, and the staple can be slid longitudinally of the ligament to come into engagement with the bar 31 and then to apply any required initial tension to one end of the ligament (assuming the other end has already been anchored), and then the staple is driven into the underlying bone.

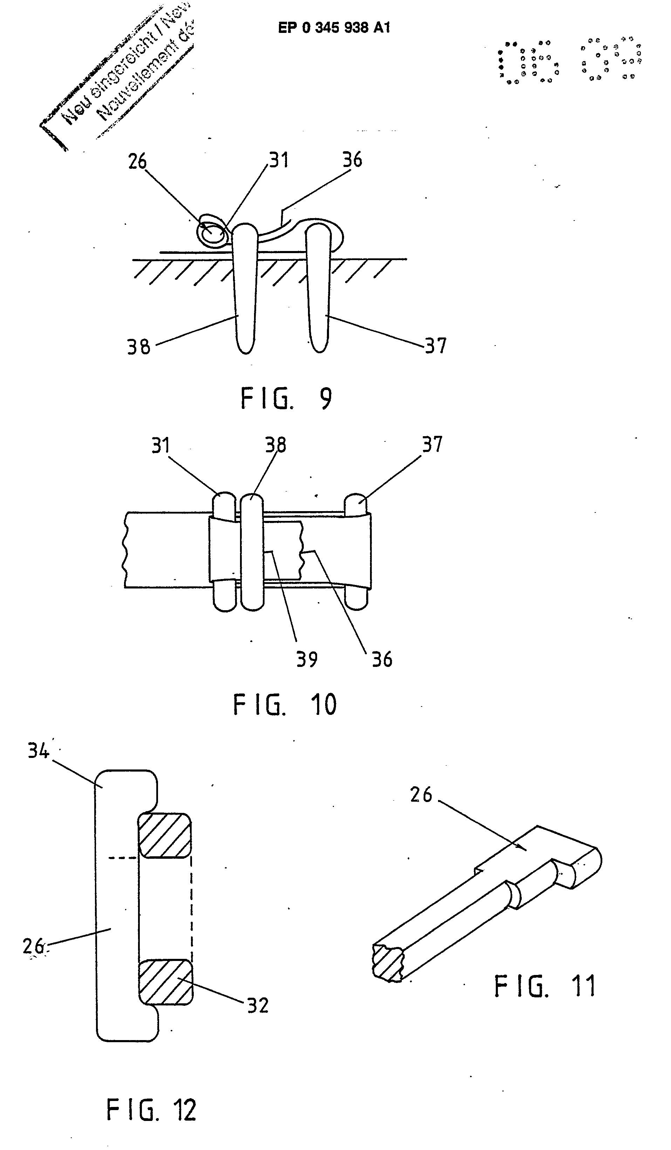

Figure 6 shows simple mechanical abutment between staple 32 and bar 31, and this engagement may be made a more co-operative and secure engagement by providing the bar with inturned shaped ends 34, as shown in Figure 12.

If a more strengthened arrangement is required, the mechanical fixation may be completed by an arrangement as shown in Figures 7 and 8. In this arrangement, staple 32 is driven into the bone and adjacent to bar 26, but the free end 35 of the ligament 30 is first folded over the bar 26 and threaded through the underside of the cross piece of the staple 32, prior to the staple being driven into the bone. This provides a more secure fastening of the end portion of the ligament to the bone.

A still further strengthened arrangement may be adopted as shown in Figure2 9 and 10. In this arrangement, bar 31 is taken through a transverse tubular portion 26 near the free end 36 of the ligament, a first staple has its cross piece taken through a further adjacent tubular portion 26, or else the ligament is folded over it, and then a second staple 38 is driven into the bone while trapping at least two superposed layers of ligament below it. In fact, in the arrangement shown in Figures 9 and 10, the free end 36 is tucked under the cross pice 39 of the second staple 38, prior to the latter being driven into position, and this therefore provides three superposed layers of ligament which can be trapped below the cross piece 39 of second staple 38.

Figure 11 shows one preferred profile of anchoring bar 26 having a rebate 40 which enables the end portion of the ligament to be laid onto it.

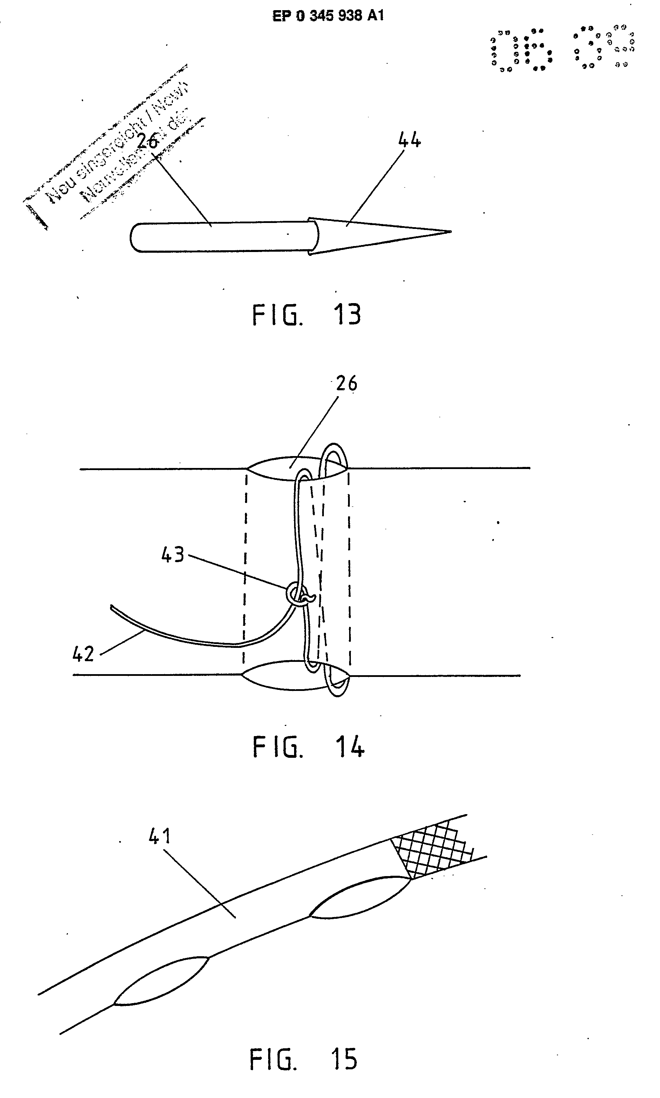

If it is required to carry out additional ligament reconstruction, such as extra articular reconstruction of ligament or tissue e.g. medial co-lateral ligaments of the knee or lateral extra articular reinforcement, the ligament shown in Figure 3 or 5 may be extended by provision of an additional length 41, as shown in Figure 15. This additional length 41 may project from the passage in one of the bones, shown in Figure 2, and can then be externally laid over the knee bones and anchored in position.

To facilitate entry of any particularly selected elongate anchoring element e.g. a cross piece of a staple, or anchoring bar 26, means may be provided to facilitate temporary enlargement of the entrance mouth to the tubular portion at one or both ends thereof, as shown in Figure 14. This comprises a cord or drawstring 42 which is looped through tubular portion 26 and then taken through a guide, such as slip knot 43, and which can be tightened so as to draw the ends of the tubular portion 26 towards each other and thereby open the mouths at the ends. The cord may be cut subsequently, after introduction of the anchoring element into it. This will facilitate the surgeon in his placement of the elongate anchoring element in tubular portion 26.

Figure 13 shows a detachable guide head 44 which may be provided on one end of an anchoring bar 26, which can also facilitate threading of the bar through the transverse tubular portion or passage 26.

Each transverse tubular portion 26 in which an elongate anchoring element 15, 31 can be received is a strengthened section of the ligament, by being formed as a dense weave section, and each adjacent strengthened section 27 is also a dense weave section, but is further strengthened by being formed as a closure.

## Claims

1. A prosthetic ligament made of woven implantable material and which can be introduced into a passage formed in a pair of adjoining bones of a bone joint, there being enlarged entrances formed at each end of the passage to receive bone plugs for closing the entrances after the ligament has been introduced into the passages and the entrances, and the ligament comprising:

a main tubular portion to be located in said passage and having its axis extending generally longitudinally of the ligament; and,

end portions each intended to be located in a respective entrance and to project therefrom for securement to the respective bone by mechanical fixation:

in which at least one end portion of the ligament comprises:

a tubular portion which extends transversely of said longitudinal axis and which is open at each end so as to receive an elongate anchoring element of a mechanical fixation arrangement which is operative to make a mechanical fixation of the end portion to the respective bone; and,

a strengthened section adjacent to the transverse tubular portion and arranged to withstand tensile load which may be applied in use to the ligament by the mechanical fixation arrangement.

2. A prosthetic ligament according to Claim 1, and in combination with a mechanical fixation arrangement including a U-shaped staple having substantially parallel spikes which can be driven into the underlying bone.

3. A prosthetic ligament according to Claim 2, in which the staple has its cross piece receivable by the tubular portion so as to form said elongate anchoring element.

4. A prosthetic ligament according to any one of Claims 1 to 3, in which said one end portion is provided with a pair of transverse open ended tubular portions, each intended to receive a respective elongate anchoring element.

5. A prosthetic ligament according to Claim 4, in which said one end portion is formed with an alternating serious of transverse tubular portions and dense weave sections, whereby a double staple mechanical fixation can be obtained with the staples being introduced at any desired positon along the length of the end portion, by introduction into any selected ones of the tubular portions available.

6. A prosthetic ligament according to Claim 2, in which the mechanical fixation arrangement comprises an anchoring bar which forms said elongate anchoring element and which can be introduced into a respective transverse tubular portion, and a U-shaped staple which can fit over the end portion of the ligament and adjacent to the anchoring bar.

7. A prosthetic ligament according to Claim 6, in which the anchoring bar has inturned ends which can hold the staple therebetween.

8. A prosthetic ligament according to Claim 6 or 7, in which the bar is formed with a rebate onto which the end portion of the ligament can be laid.

9. A prosthetic ligament according to any one of Claims 6 to 8, in which the mechanical fixation arrangement comprises first and second staples, one of which can be used to hold down one layer of the end portion, and the free end of which can then be folded back over the cross piece of the spike, and the other staple of which can then be driven into the underlying bone and trapping the two layers of end portion therebetween, and with said further staple being located adjacent to a respective anchoring bar located in its transverse tubular portion.

10. A prosthetic ligament according to Claim 9, in which the free end of the end portion is taken under the cross piece of the further staple so that three superposed layers of the end portion of the ligament can be trapped under the cross piece of said further staple.

11. A prosthetic ligament according to any one of Claims 1 to 10, including an additional length of ligament attached thereto, and which can project from the passage in one of the bones, and which can then be externally applied to the bone joint during additional ligament reconstruction.

12. A prosthetic ligament according to any one of Claims 1 to 11, including a cord arrangement looped through one of the tubular portions and taken through a guide, and which can be tightened so as to draw the ends of the tubular portion towards each other and thereby open up the entry mouths to the tubular portion to facilitate insertion of a respective elongate anchoring element.

13. A prosthetic ligament according to any one of Claims 1 to 12, in which the elongate anchoring element is provided with a detachable guide head which facilitates insertion of the anchoring element into its respective tubular portion.

14. A prosthetic ligament according to any one of Claims 1 to 13, in which the strengthened section of said one end portion is formed by a dense weave section.

15. A prosthetic ligament according to Claim 14, in which the ligament is a woven ligament having at least the main tubular portion formed as a mock leno weave.

16. A method of implanting a prosthetic ligament made of woven implantable material in a pair of adjoining bones of a bone joint, in which the prosthetic ligament comprises a main tubular portion having its axis extending generally longitudinally of the ligament, and end portions attached to the main tubular portion, and in which the method comprises:

forming a passage in a pair of adjoining bones of the bone joint, and also forming enlarged entrances at each end of the passage;

introducing the ligament into the passage and the entrances, so as to locate the main tubular portion in the passage and to locate each end portion in a respective entrance from which it projects for securement to the respective bone by mechanical fixation, and at least one of the end portions of the ligament being provided with a tubular portion which extends transversely of the longitudinal axis and which is open at each end, and a strengthened section adjacent to the transverse tubular portion;

securing said one end portion of the ligament to its respective bone by introducing an elongate anchoring element of a mechanical fixation arrangement into said tubular portion, and operating the mechanical fixation arrangement so as to make a mechanical fixation of said one end portion to the respective bone; and,

introducing bone plugs to close said entrances after the ligament has been introduced into the passage and the entrances.

# FIG. 1

# FIG. 2

FIG. 2a

FIG. 2b

FIG. 2c

FIG. 3

FIG. 4a

FIG. 4b

FIG. 4c

FIG. 4d

30  33  32  26  26

21  23  27  33  31  27

EP 0 345 938 A1

FIG. 5

EP 0 345 938 A1

FIG. 6

FIG. 7

FIG. 8

26   31   36

38   37

FIG. 9

31   38   37

39   36

FIG. 10

34

26

32

FIG. 12

26

FIG. 11

26

44

FIG. 13

26

43

42

FIG. 14

41

FIG. 15

European Patent Office

**PARTIAL EUROPEAN SEARCH REPORT**

which under Rule 45 of the European Patent Convention shall be considered, for the purposes of subsequent proceedings, as the European search report

Application number

EP 89 30 4347

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A-2 039 220 (QUEEN'S UNIVERSITY) | | A 61 F 2/08 |
| | * Page 2, lines 98-105; figure 3 * | 1 | |
| | -- | | |
| A | EP-A-0 051 954 (HOMSY) | | |
| | * Page 9, lines 17-20; figure 5 * | 1 | |
| | -- | | |
| A | EP-A-0 260 787 (GORE) | | |
| | * Column 3, lines 22-34; figure 2 * | 1 | |
| | -- | | |
| A,D | EP-A-0 126 520 (BAHAA BOTROS) | | |
| | * Page 8, lines 7-17; figure 1 * | 1 | |
| | ----- | | |

**TECHNICAL FIELDS SEARCHED (Int. Cl.4)**

A 61 F

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-15

Claims searched incompletely:

Claims not searched: 16

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 28-08-1989 | MOERS |